Europäisches Patentamt

European Patent Office

Office européen des brevets

(19)

(11) Publication number : **0 481 684 A2**

(12) # EUROPEAN PATENT APPLICATION

(21) Application number : 91309335.7

(22) Date of filing : 10.10.91

(51) Int. Cl.⁵ : **A61B 5/0464,** A61N 5/06,
**A61N 1/40,** A61N 5/02,
**A61N 1/362**

(30) Priority : 19.10.90 US 601241
19.10.90 US 601249

(43) Date of publication of application :
22.04.92 Bulletin 92/17

(84) Designated Contracting States :
AT BE CH DE DK ES FR GB GR IT LI LU NL SE

(71) Applicant : ANGELASE, Inc.
13000 Highway 55
Plymouth, Minnesota 55441 (US)

(72) Inventor : Svenson, Robert H. M. D.
2327 Hopedale Avenue
Charlotte, NC 28207 (US)
Inventor : King, Wendell
7 Island View Lane
North Oaks, Minnesota 55127 (US)

(74) Representative : Parr, Ronald Edward R.E. Parr
& Co.
Colman House Station Road
Knowle Solihull West Midlands B93 0HL (GB)

(54) Location and ablation of an active site of ventricular tachycardia.

(57) An electrophysiologically guided arrhythmia ablation system for ventricular tachycardia or other arrhythmias. The system combines a system for recording the electrical activation time of various parts of the heart for locating an active site of the arrhythmia, with a system for delivering energy for ablation of the arrhythmia. The system has the capability of processing local myocardial electrical activation data and triggering the ablation energy source when at the active site or inhibiting the release of potentially harmful energy when the device is not at an active site. The process includes identification of an active site which occurs during the 20-80%, preferably 35-50%, time period of a diastolic interval. The diastolic interval is monitored by appropriate electrical connections between the heart and a monitor. A mapping device also connects to the monitor which maps for indicating an active site. The active site is then a site for preferred placement of an electrode for a defibrilator. A timing system connects to the monitor, indicates when an active site is located in the 20-80%, preferably 35-50%, of the diastolic interval, and also connects to a laser system for firing the laser system to ablate the active site. Other ablate energies can be utilized including RF, microwave or DC energy.

FIG. 1

Jouve, 18, rue Saint-Denis, 75001 PARIS

## BACKGROUND OF THE INVENTION

1. Definitions :

A. VT - Ventricular tachycardia.
B. Active Site - Critical site to deliver ablation energy to cure VT identified by electrical activations. The energy must be delivered to the heart tissue to ablate the heart tissue.
C. Diastolic - That period of time between two QRS complexes of the electrocardiogram.
D. Ablation - The delivery of destructive energy to the cardiac tissues containing the active site.

2. Field of the Invention -

the present invention pertains to medical apparatus and processes and, more particularly, pertains to cardiac electrophysiology, for example, ablation of cardiac arrhythmias or modification of the electrical properties of the myocardium. In one aspect the present invention also pertains to a process for identification of an active site of origin of ventricular tachycardia and for electrode attachment of an endocardial defibrilator lead, as well as a catheter system for ablation of the active site of the ventricular tachycardia.

3. Description of the Prior Art -

In existing technology, the recognition of the site of the origin of the arrhythmias and the ablation function are performed separately. For ventricular tachycardia, there has been no consensus of opinion as to what electrical activation times constitute the "site of origin". Furthermore, the ablation energy source, whether DC current shock, radio frequency, microwave, or laser, has to be separately redirected by visual means to the site of suspected origin of the arrhythmia.

Arrhythmia ablation is currently performed during open heart surgery or through catheters directed percutaneously through the heart. During the surgical approach, either a hand-held electrical mapping probe or a computerized array of electrodes acquire electrical activation data seeking the site of origin of the arrhythmia. In the percutaneous catheter based approach, a catheter with recording electrodes is positioned in the heart under fluoroscopic guidance.

Following acquisition of electrical activation data, ablation energy is then later delivered by hand-held probes or catheters either in the operating room or in the cardiac catheterizational laboratory.

In the prior art, the process for identification of the "site of origin" of the arrhythmia was performed with electrical recording procedures designed to map the spread of electrical activation in the heart looking for the site of earliest electrical activation (site of origin). This procedure is carried out by sequentially moving a handheld electrical recording probe or catheter over the heart and recording the time of arrival of the electrical impulse to that location. This process turned out to be a long and tedious procedure.

Prior art mapping procedures also include a sock multiple electrode array (epicardial), a balloon endocardial electrode array, a single hand-held mapping probe, or a multiple electrode catheter (endocardial) inside a chamber of the heart. These procedures require a skilled surgeon and cardiac electrophysiologist.

The particular concern was the preferred location for an electrode for an endocardial defibrilator. It was not an easy procedure to determine the preferred placement at the active site.

The prior art mapping procedures are capable of reconstructing the spread of electrical activation in the heart, but do not in themselves identify the "active site" of the arrhythmia, can be time consuming, and are separate functions from the prior art ablation procedures.

It has now been found, according to the present invention , that the disadvantages of the prior art can be overcome by identifying a particular time zone of electrical activation during the diastolic interval of the arrhythmia where ablation energy can be delivered with a high probability of successful cure.

## SUMMARY OF THE INVENTION

The general purpose of the present invention is apparatus and process for identification of the critical site to attach an electrode for an endocardial defibrilator to control cardiac arrhythmia, and to delivery therapeutic ablation energy to cardiac arrhythmia. For ventricular tachycardia, the process involves identification of a site activating during the 20-80%, preferably 35-50% of the electrical diastolic period as referenced to the body surface electrocardiogram. The electrode can be either an endocardial electrode or an epicardial electrode as may be requested.

According to one aspect of the present invention, there is provided a process for identification of a site or sites for attachment of an electrode for a defibrilator of diastolic activation during ventricular tachycardia, including the steps of measuring the diastolic interval of a heart, mapping heart tissue for an active site, identifying the active site in the 20-80%, preferably 35-50%, time period of the diastolic interval. The electrode can be, for example either an endocardial electrode or an epicardial electrode.

According to another aspect of the present invention, there is provided a process for identification of an active site of diastolic activation during ventricular tachycardia including the steps of monitoring an ECG signal, monitoring the local diastolic activation time in relation to the ECG signal, mapping heart tissue in a chamber of the heart for an active site, and identifying the active site in the 20-80%, preferably 35-50%, time period of the diastolic interval and initiating the delivery of ablation energy to the active site. There may be more than one active site.

The system includes the electrical activation data, whether from a hand-held mapping probe or computerized electrode array in the operating room or with an electrode catheter by percutaneous approach. The system includes recognition of the appropriate electrical markers of the active site. The system also includes an interface with the ablation energy source. The first part guides the delivery of the ablation energy. This differs from old procedures in that the two functions have now been integrated into a system. The new aspect of the system includes a new recognition of the appropriate electrical markers of the active site of ventricular tachycardia coupled to an automated ablation delivery system. The problems solved by this invention include overcoming the unreliability of visually directed ablation with separately obtained sites of local activation. This is particularly important since the critical areas requiring ablation energy may require a reproducibility of location within 0.5 centimeters or less. For ventricular tachycardia, the critical ablation of the active site defined in electrical activation terms encompasses a critical range of electrical activation time within electrical diastole as viewed from the surface ECG, i.e., 20-80%, preferably 35-50%, of the time between QRS complexes of the arrhythmia. This critical range has not previously been identified, nor has this activation time been coupled to the ablation source.

The present invention is based on our discovery that active sites occur during the 20-80%, preferably 35-50%, time period of a diastolic interval. With this recognition, the appropriate ablation of the active site can then be undertaken to eliminate the cells causing the VT condition as the ablation energy can automatically be delivered through the mapping device, or in the alternative, a second device adjacent to the mapping device.

Other significant aspects and features are that potentially dangerous energy cannot be delivered to normal heart muscle; therapeutic ablation energy can be delivered in spite of the transient nature of the arrhythmia; and the device overcomes serious problems imposed by movement of the mapping device while a physician is trying to visually access the activation time and make an independent judgment about energy delivery.

Another significant aspect and feature of the present invention is the recognition of a time zone of 160-50 milliseconds before the onset of a QRS complex in which active sites occur during VT.

A further significant aspect and feature of the present invention is that a site of origin for VT is now recognized as the active site.

The present invention provides a process for identification of active sites for attachment of an electrode for an endocardial defibrilator, and for later ablation of the cells at the active site through the delivery of ablation energy. The invention is based on knowledge of the active site of ventricular tachycardia; the knowledge of ablation effectiveness; and the coupling of the activation time to the energy ablation source.

In a preferred form the present invention provides ablation apparatus which is automatic in that when an active site is sensed by the ECG signal processing for a preferred 35-50% range in a 20-80% range of the diastolic interval, energy is automatically switched to the energy delivery structure so that the electrophysiologist is not distracted during a mapping procedure . This automatic switching of energy enhances accurate delivery of energy to the mapped active site in a relatively instantaneous time period.

## BRIEF DESCRIPTION OF THE DRAWINGS

There are now described, by way of example and with reference to the accompanying drawings, preferred embodiments of the processes and apparatus of the present invention.

In the drawings:

FIG. 1 illustrates schematically apparatus for a process of identification of an active site and attachment of an electrode for a defibrilator;

FIG. 2 illustrates schematically apparatus for a process of identification of an active site, as well as ablation of the active site; and

FIG. 3 illustrates audio visual apparatus for indication of an active site.

## DESCRIPTION OF THE PREFERRED EMBODIMENTS

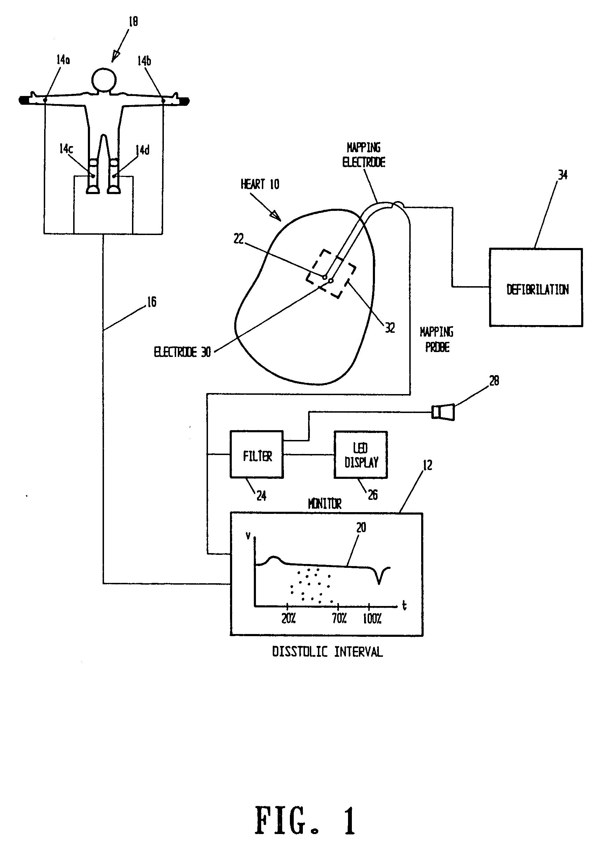

FIG. 1 illustrates apparatus for a process of identification of an active site in a heart(10)of a patient (18). The apparatus includes a monitor (12) and ECG electrodes (14a-14d) connected by a cable (16) from the patient (18) to the monitor 12. A mapping electrode 22 for mapping of an active site or sites also connects to the monitor 12. A display 20 is shown on the monitor 12.

A filter 24 can connect to the mapping electrode lead 22, and an illuminating display 26 such as an LED connects to the filter to indicate an active site. An electrical signaling device (28),for example a tone generator, can also connect to the filter to indicate an active site.

Active sites are indicated between the 20-80%, preferably 35-50%, time period of the diastolic interval on the display.

Once an active site is located, the site is appropriate for placement of an electrode for a defibrilator 34. The endocardial electrode 30 can also be the mapping electrode, one and the same as illustrated in the box 32 in the dashed lines for purposes of illustration only and not to be construed or limiting of the present invention. Any suitable defibrilation can be utilized corresponding to the appropriate type of electrode.

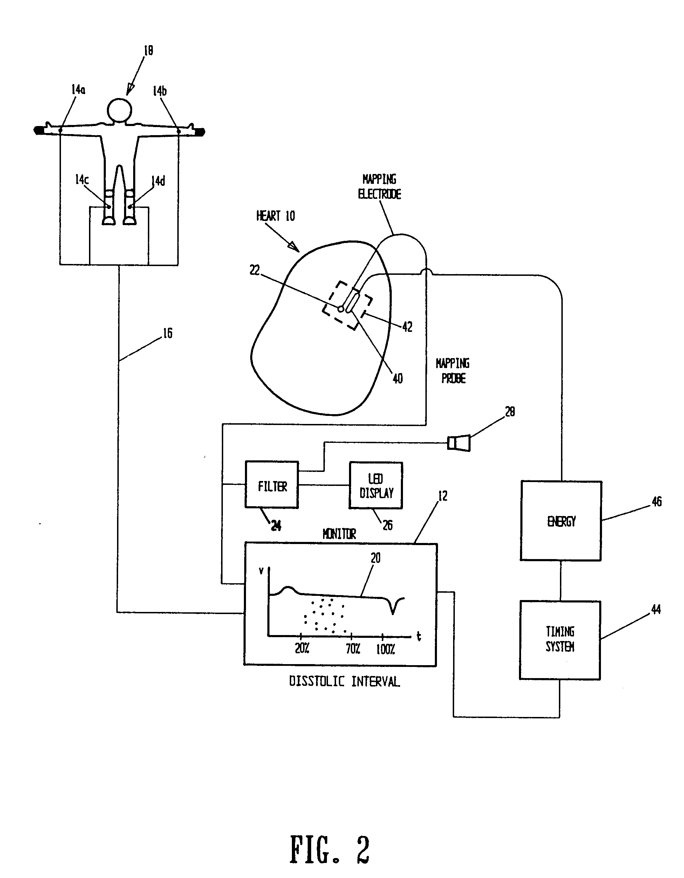

FIG. 2 illustrates apparatus for a process of identification of an active site in a heart 10 of a patient 18. The apparatus includes a monitor 12 and ECG electrodes 14a-14d connected by a cable 16 from the patient 18 to the monitor 12. A mapping electrode 22 for mapping of an active site or sites also connects to the monitor 12. A display 20 is shown on the monitor 12. A diastolic interval is obtained from the ECG signal between the QRS complexes.

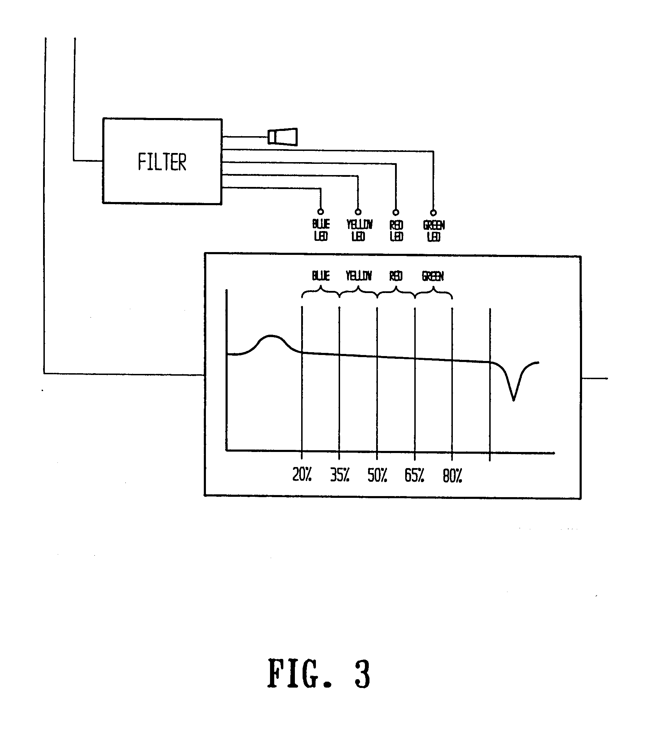

A filter 24 can connect to the mapping electrode lead 22, and an illuminating display 26 such as an LED connects to the filter to indicate an active site. An electrical signaling device 28, for example a tone generator, can also connect to the filter to indicate an active site. A plurality of LEDs and tones can be connected to the filter to indicate the degree of the active site as later described in FIG.3.

Active sites are indicated between the 20-80%, preferably 35-50%, time period of the diastolic interval as shown on the display.

A timing system 44 connects to the monitor 12 to switch a laser or other energy source 46 during sensing of an active site during the 20-80%, preferably 35-50%, time period of the diastolic interval and power an ablation device 40 with laser or other energy source for ablation of the VT cells at the active site. Other energy sources can, for example include RF, microwave or DC energy to ablate the active site. The mapping electrode can also be used to deliver the RF, microwave or DC energy in lieu of using a separate ablation lead such as which would be required for delivery of laser energy. The ablation device 40 and mapping electrode 22 can position in the same housing 24 such as at the end of a catheter. The mapping electrode 22 and the ablation lead 40 can be one and the same.

It is within the teachings of this disclosure that the mapping and the energy delivery structure for ablation of the heart tissue can be the same device, such as a catheter by way of example and for purposes of illustration only, and not to be construed as limiting of the present invention. As an example, the mapping electrode could be used to deliver the RF, microwave or DC energy. The laser delivery structure, such as a fiber optic and lens, could be arranged adjacent to the mapping electrodes or surrounded by the mapping electrodes in an appropriate catheter structure.

It is preferred that the mapping electrode and the ablation device are within the same structure such as a catheter as indicated by a box 42 in dashed lines.

FIG. 3 illustrates audio visual apparatus for indication of an active site including active site time zones corresponding to a plurality of different colored LEDs connected to the filter by way of example and for purposes of illustration only, and not to be construed as limiting of the present invention. A corresponding time zone can be associated with each corresponding LED and in the event the electrophysiologist is unable to view the display or the LEDs, the electrophysiologist would be able to listen to the audible tones corresponding to the time zones. An example is set forth in Table 1. All other electrical circuitry corresponds to that as previously described in FIG. 2.

### Table 1

| Range | LED Color | Musical Tone |
|-------|-----------|--------------|
| 20-35% | Blue | B |
| 35-50% | Yellow | C |
| 50-65% | Red | D |
| 65-80% | Green | E |

## MODE OF OPERATION

The steps for the process of identification of an active site for VT and for attachment of an electrode for a defibrilator are performed by appropriate medical personnel in accordance with the description of the preferred embodiments above. The recognition of the active sites in the preferred 35-50% window or broadly, the 20-80% window, of the diastolic is the present invention. The teachings of the present invention can be utilized, for example for either an endocardial electrode or an epicardial electrode for a defibrilator.

## Claims

1. Apparatus for identification of an active site of diastolic activation during ventricular tachycardia, characterised in that the apparatus comprises:
   (a) measuring means for measuring a diastolic interval of a heart;
   (b) mapping means for mapping tissue of the heart thereby to identify an active site in the 20% - 80% time period of said diastolic interval; and
   (c) an electrode for treatment of the active site.

2. Apparatus according to Claim 1, wherein said electrode is an endocardial electrode.

3. Apparatus according to Claim 1, wherein said electrode is an epicardial electrode.

4. Apparatus according to any of the preceding claims, which includes an energy source and means operatively connecting the energy source to the mapping means, whereby the energy source can be activated once the active site has been identified.

5. Apparatus for location and ablation of an active site of ventricular tachycardia, characterised in that the apparatus comprises:
   (a) a catheter;
   (b) a mapping electrode at one end of said catheter and connected to a signal processing and display;
   (c) an ablation means at said end of said catheter;
   (d) said signal processing and display connected to ECG signals for processing and displaying a diastolic interval;
   (e) an energy source connected to said ablation means; and
   (f) a switch means connected between said signal processing and display means and said energy source to switch energy during a 20 - 80% diastolic interval.

6. Apparatus according to Claim 4 or 5, wherein said energy source is a laser, RF energy, microwave energy or DC energy.

7. Apparatus according to Claim 5 or 6, wherein said interval is 20 - 80% of the diastolic interval.

8. Apparatus according to any of the preceding claims, wherein said interval is 35 - 50% of the diastolic interval.

9. Apparatus according to any of Claims 5 to 8 wherein said mapping electrode and said ablation means are the same electrodes.

10. Apparatus according to any of Claims 5 to 9, wherein said mapping electrode and said ablation means including a laser optic means are immediately adjacent to each other in said catheter.

11. Process for identification of an active site of diastolic activation during ventricular tachycardia (VT) for electrode attachment for a defibrilator comprising the steps of:
    (a) measuring a diastolic interval of a heart;
    (b) mapping heart tissue for an active site;
    (c) identifying the active site in the 20% - 80% time period of said diastolic interval; and
    (d) attaching an electrode at the active site for a defibrilator.

12. Process for ablation of an active site comprising the steps of:
    (a) measuring a diastolic interval of a heart;
    (b) mapping heart itssue for an active site;
    (c) identifying the active site in the 20% - 80% time period of said diastolic interval; and
    (d) switching an energy course to power an ablation means adjacent said mapping electrode to ablate the active site.

FIG. 1

**FIG. 2**

FILTER

BLUE
LED

YELLOW
LED

RED
LED

GREEN
LED

BLUE YELLOW RED GREEN

20%    35%    50%    65%    80%

FIG. 3